# EUROPÄISCHE PATENTSCHRIFT

(11) **EP 1 123 698 B1**
(45) Veröffentlichungstag und Bekanntmachung des Hinweises auf die Patenterteilung: **26.04.2006**
(21) Anmeldenummer: 01100871.1
(22) Anmeldetag: 16.01.2001
(51) Int. Cl.: A61K 8/37

(54) **Wässrige oder wässrig-alkoholische Körperreinigungsmittel enthaltend Oligoester**
Aqueous or aqueous-alcoholic personal cleansing compositions containing oligoesters
Compositions nettoyantes aqueuses ou hydroalcooliques pour la peau, contenant des oligoesters

(30) Priorität: 26.01.2000 DE 10003137
(43) Veröffentlichungstag der Anmeldung: 16.08.2001
(73) Patentinhaber: Clariant Produkte (Deutschland) GmbH, 65929 Frankfurt am Main (DE)
(72) Erfinder: Löffler, Matthias, Dr., 65527 Niedernhausen (DE); Mulitze-Kleinheyer, Vera, 65926 Frankfurt am Main (DE)

(56) Entgegenhaltungen:
- EP-A- 0 763 354
- FR-A- 2 760 643
- FR-A- 2 781 233
- US-A- 4 785 060
- CHEMICAL ABSTRACTS, vol. 130, no. 1, 4. Januar 1999 (1999-01-04) Columbus, Ohio, US; abstract no. 5129, "Optimised soil release polymers. Basic effects, detergency benefits, and interactions with detergent ingredients" XP002169316 & LANG,FRANK-PETER: CHIM. OGGI, Bd. 16, Nr. 9, 1998, Seiten 14-18,

## Beschreibung

Die Erfindung betrifft die Verwendung von wässrigen oder wässrig-alkoholischen Körperreinigungsmitteln zur milden Reinigung und Pflege der Haut, gekennzeichnet durch den Gehalt an Oligoester, enthaltend Dicarbonsäureeinheiten und Dioleinheiten (Glykol-, Alkylglykol- und/oder Polyalkylenpolyglykoleinheiten), wie beansprucht in den Ansprüchen.

Körperreinigung und -pflege in zwei Schritten ist zeitraubend, so dass von vielen Verbrauchern Mittel mit reinigender und pflegender Wirkung zugleich, bevorzugt werden.
Eine Vielzahl von kosmetischen Produkten versuchen diesem Anspruch gerecht zu werden.

In US 5,612,307 werden wässrige, flüssige Körperreinigungsmittel beansprucht, die neben üblichen Tensidsystemen eine pflegende Komponente aus der Gruppe der Siliconöle, Fette, Öle, Wachse, hydrophoben Pflanzenextrakte, Fettsäuren, Alkoholen, Ester, Lipiden und/oder Phospholipiden, enthalten.
In WO 94/03152 werden Duschgels beansprucht, bestehend im wesentlichen aus einem Tensid, Siliconöl und einem kationischen Polymer. In EP 0 763 354 werden kosmetisch W/O-Emulsionen offenbart, die Oligoester, erhalten aus polyvalenten Alkoholen und Dicarbonsäuren, enthalten.
Unbefriedigend ist, dass pflegende und feuchtigkeitsspendende Komponenten nicht in ausreichender Menge in wässrige Reinigungsmittelformulierungen eingearbeitet werden können. Ein weiteres Problem ist, dass sich wässrige Dispersionen aus Tensidsystemen und feuchtigkeitsspendenden und pflegenden Komponenten im Laufe der Zeit separieren und somit wenig lagerstabil sind.

Es bestand daher die Aufgabe, neue hautpflegende Körperreinigungsmittel zu entwickeln, die reinigende und pflegende Wirkung in sich vereinen und die frei sind von den oben genannten Nachteilen.

Gegenstand der Erfindung ist die Verwendung von wässrigen oder wässrig-alkoholischen Körperreinigungsmitteln ausgenommen Haarbehandlungsmittel, enthaltend Oligoester, die durch Kondensation von einer oder mehreren Dicarbonsäuren oder deren Estern und einem oder mehreren mehrwertigen Alkoholen erhalten werden, 5 bis 70 Gew.-% an Tensiden enthalten, schäumend sind, und keine W/O- oder O/W- Emulsionen darstehen, zur Reinigung der Haut.

Diese Oligoester werden vorzugsweise erhalten durch Polykondensation von einer oder mehreren aromatischen Dicarbonsäuren oder deren Ester mit Ethylenglykol und/oder Propylenglykol. Gegebenenfalls können diese Ester auch Polyethylenglykol, Polypropylenglykol, Sulfoisophthalsäure, Sulfobenzoesäure, Isethionsäure, C₁-C₄-Alkohole, oxalkylierte C₁-C₂₄-Alkohole, oxalkylierte C₆-C₁₈₋Alkylphenole und/oder oxalkylierte C₈-C₂₄-Alkylamine als Monomere enthalten.

Insbesondere bevorzugt ist die Verwendung von solchen Oligoestern, die erhalten werden durch Polykondensation von
a) 40 bis 52, vorzugsweise 45 bis 50 Mol-% einer oder mehrerer Dicarbonsäuren oder deren Ester,
b) 10 bis 40, vorzugsweise 20 bis 35 Mol-% Ethylenglykol und/oder Propylenglykol,
c) 3 bis 20, vorzugsweise 10 bis 15 Mol-% Polyethylenglykol,
d) 0 bis 10 Mol-% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 Mol-% eines oder mehrerer Polyole mit 3 bis 6 Hydroxylgruppen.

Als Komponente a) zur Herstellung der Copolyester eignen sich beispielsweise Terephthalsäure, Phthalsäure, Isophthalsäure sowie die Mono- und Dialkylester mit C₁-C₆-Alkoholen, wie Dimethylterephthalat, Diethylterephthalat und Di-n-propylterephthalat. Weitere Beispiele für Verbindungen, die als Komponente a) zur Herstellung der Polyester eingesetzt werden können, sind Oxalsäure, Bernsteinsäure, Glutarsäure, Adipinsäure, Fumarsäure, Maleinsäure, Itakonsäure, sowie die Mono- und Dialkylester der Carbonsäuren mit C₁-C₆-Alkoholen, z.B. Oxalsäurediethylester, Bernsteinsäurediethylester, Glutarsäurediethylester, Adipinsäuremethylester, Adipinsäurediethylester, Adipinsäure-di-n-butylester, Fumarsäureethylester und Maleinsäuredimethylester. Sofern die in Betracht kommenden Dicarbonsäuren Anhydride bilden können, sind auch die Anhydride der mindestens 2 Carboxylgruppen aufweisenden Carbonsäuren als Verbindung der Komponente a) zur Herstellung der Oligoester geeignet, z.B. Maleinsäureanhydrid, Phthalsäureanhydrid oder Bernsteinsäureanhydrid. Besonders bevorzugt werden als Verbindung der Komponente a) Terephthalsäure, Phthalsäure, Isophthalsäure sowie deren Dimethyl-, Diethyl-, Dipropyl- und Dibutylester eingesetzt. Es ist selbstverständlich möglich, Mischungen verschiedener Carbonsäuren oder verschiedener Ester einzusetzen. Ebenso kann man auch beispielsweise Mischungen aus Carbonsäuren und Ester oder Mischungen aus Carbonsäuren und Anhydriden bei der Kondensation verwenden.

Als Komponente c) verwendet man Polyethylenglykole mit Molmassen von 500 bis 5000, vorzugsweise von 1000 bis 3000.

Als Komponente d) zur Herstellung der Oligoester kommen wasserlösliche Anlagerungsprodukte von 5 bis 80 mol mindestens eines Alkylenoxids an 1 mol C₁-C₂₄-Alkohole, C₆-C₁₈-Alkylphenole oder C₈-C₂₄-Alkylamine in Betracht. Bevorzugt sind Monomethylether von Polyethylenglykolen. Als Alkylenoxide zur Herstellung der Verbindungen der Komponente d) verwendet man vorzugsweise Ethylenoxid sowie Mischungen aus Ethylenoxid und Propylenoxid. Außerdem eignen sich Mischungen aus Ethylenoxid zusammen mit Propylenoxid und/oder Butylenoxid, Mischungen aus Ethylenoxid, Propylenoxid und Isobutylenoxid oder Mischungen aus Ethylenoxid und mindestens einem Butylenoxid. Diese wasserlöslichen Anlagerungsprodukte der Alkylenoxide sind Tenside. Falls zu ihrer Herstellung Mischungen von Alkylenoxiden verwendet wurden, so können sie die Alkylenoxide in Blöcken oder auch in statistischer Verteilung enthalten.

Geeignete Alkohole, die alkoxyliert werden, sind beispielsweise Octylalkohol, Decylalkohol, Laurylalkohol, Myristylalkohol oder Stearylalkohol, insbesondere aber Methanol, sowie die nach dem Ziegler-Verfahren erhältlichen Alkohole mit 8 bis 24 C-Atomen oder die entsprechenden Oxoalkohole. Von den Alkylphenolen haben insbesondere Octylphenol, Nonylphenol und Dodecylphenol Bedeutung. Von den in Betracht kommenden Alkylaminen verwendet man insbesondere die C₁₂-C₁₈₋Monoalkylamine.
Als Polyole (Komponente e) kommen beispielsweise in Frage Pentaerythrit, Trimethylolethan, Trimethylolpropan, 1,2,3-Hexantriol, Sorbit, Mannit und Glycerin.

Die Synthese der erfindungsgemäßen Oligoester erfolgt nach an sich bekannten Verfahren, indem die Komponenten a, b und c sowie gegebenenfalls d und e unter Zusatz eines Katalysators zunächst bei Normaldruck auf Temperaturen von 160 bis ca. 220°C erhitzt werden. Dann wird die Reaktion im Vakuum bei Temperaturen von 160 bis ca. 240°C unter Abdestillieren überschüssiger Glykole fortgesetzt. Für die Reaktion eignen sich die bekannten Umesterungs- und Kondensationskatalysatoren des Standes der Technik, wie beispielsweise Titantetraisopropylat, Dibutylzinnoxid oder Antimontrioxid/Calciumacetat. Bezüglich weiterer Einzelheiten zur Durchführung des Verfahrens wird auf EP 442 101 verwiesen.

Besonders geeignet sind auch die aus EP 241 985 bekannten Polyester, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁-C₄-Alkylgruppen endgruppenverschlossen sind, die in EP 253 567 beschriebenen schmutzablösevermögenden Polymere mit einer Molmasse von 900 bis 9000 g/mol aus Ethylenterephthalat und Polyethylenoxidterephthalat, wobei die Polyethylenglykol-Einheiten Molgewichte von 300 bis 3000 g/mol aufweisen und das Molverhältnis von Ethylenterephthalat zu Polyethylenoxidterephthalat 0,6 bis 0,95 beträgt, und die aus EP 272 033 bekannten, zumindest anteilig durch C₁-C₄-Alkyl- oder Acylreste endgruppenverschlossenen Polyester mit Polypropylenterephthalat- und Polyoxyethylenterephthalat-Einheiten.

Gleichfalls bevorzugt sind Oligoester aus Ethylenterephthalat und Polyethylenoxidterephthalat, in denen die Polyethylenglykol-Einheiten Molgewichte von 750 bis 5000 g/mol aufweisen und das Molverhältnis von Ethylenterephthalat zu Polyethylenoxidterephthalat 50:50 bis 90:10 beträgt und deren Einsatz in Waschmitteln in der deutschen Patentschrift DE 28 57 292 beschrieben ist, sowie Oligoester mit Molgewichten von 15 000 bis 50 000 g/mol aus Ethylenterephthalat und Polyethylenoxidterephthalat, wobei die Polyethylenglykol-Einheiten Molgewichte von 1000 bis 10 000 g/mol aufweisen und das Molverhältnis von Ethylenterephthalat zu Polyethylenoxidterephthalat 2:1 bis 6:1 beträgt, die gemäß DE 33 24 258 in Waschmitteln eingesetzt werden können.

Ebenfalls bevorzugt sind die in DE 19 644 034 beschriebenen Oligoester der Formel 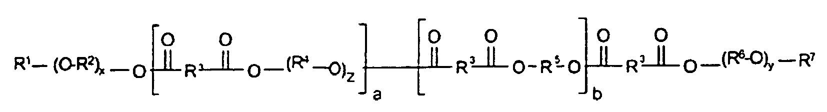 worin
- R¹ und R⁷: lineares oder verzweigtes C₁-C₁₈-Alkyl,
- R² und R⁶: Ethylen,
- R³: 1,4-Phenylen,
- R⁴: Ethylen,
- R⁵: Ethylen, 1,2-Propylen oder statistische Gemische von beliebiger Zusammensetzung von beiden,
- x und y: unabhängig voneinander eine Zahl zwischen 1 und 500,
- z: eine Zahl zwischen 10 und 140,
- a: eine Zahl zwischen 1 und 12,
- b: eine Zahl zwischen 7 und 40,
bedeuten, wobei a+b mindestens gleich 11 ist.

Bevorzugt bedeuten unabhängig voneinander
- R¹ und R⁷: lineares oder verzweigtes C₁-C₄-Alkyl,
- x und y: eine Zahl zwischen 3 und 45,
- z: eine Zahl zwischen 18 und 70,
- a: eine Zahl zwischen 2 und 5,
- b: eine Zahl zwischen 8 und 12,
a+b eine Zahl zwischen 12 und 18 oder zwischen 25 und 35. Die in DE 19 644 034 beschriebenen Oligoester werden aus Dimethylterephthalat, Ethylen- und/oder Propylenglykol, Polyethylenglykol und C₁-C₁₈-Alkylpolyethylenglykol unter Zusatz eines Katalysators zunächst durch Umesterung bei Temperaturen von 160 bis ca. 220°C und destillativer Abtrennung des Methanols bei Normaldruck und anschließender destillativer Abtrennung der überschüssigen Glykole bei Temperaturen von 160 bis ca. 240°C erhalten.

Die beschriebenen Oligoester sind in den erfindungsgemäßen wässrigen oder wässrig-alkoholischen Hautreinigungsmitteln üblicherweise in Mengen von 0,1 bis 10, vorzugsweise 0,3 bis 7, besonders bevorzugt 2 bis 5 Gew.-%, bezogen auf das fertige Mittel enthalten. Als Alkohole werden vorzugsweise Ethanol und Isopropanol eingesetzt. Diese Hautreinigungsmittel liegen nicht in Form von Öl-in-Wasser- oder Wasser-in-Öl-Emulsionen vor.

Die erfindungsgemäßen Hautreinigungsprodukte, beispielsweise Duschbäder, Duschgels, Schaumbäder, enthaltend oben genannte Oligoester oder Mischungen aus Oligoestern, können mit allen üblichen anionischen, kationischen, zwitterionischen, nicht-ionischen und amphoteren Tensiden in wässrigem oder wässrig-alkoholischem Medium kombiniert werden.
Die Gesamtmenge der in den erfindungsgemäßen Mitteln eingesetzten Tenside liegt zwischen 5 bis 70 Gew.-%, bevorzugt zwischen 10 und 40 Gew.-%, besonders bevorzugt zwischen 12 und 35 Gew.-%, bezogen auf das fertige Mittel.

Als anionische waschaktive Substanzen seien genannt: C₁₀-C₂₀-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside in den erfindungsgemäßen Mitteln liegt im Bereich von 7 bis 30 %, bevorzugt 10 bis 25 %, besonders bevorzugt 12 bis 22 %.

Geeignete kationische Tenside sind beispielsweise quartäre Ammonium-Salze wie Di-(C₁₀-C₂₄-Alkyl)-dimethyl-ammonium-chlorid oder -bromid, vorzugsweise Di-(C₁₂₋C₁₈-Alkyl)-dimethyl-ammonium-chlorid oder -bromid; C₁₀-C₂₄-Alkyl-dimethylethylammonium-chlorid oder -bromid; C₁₀-C₂₄-Alkyl-trimethyl-ammonium-chlorid oder -bromid, vorzugsweise Cetyl-trimethyl-ammonium-chlorid oder -bromid und C₂₀-C₂₂₋Alkyl-trimethyl-ammonium-chlorid oder -bromid; C₁₀-C₂₄-Alkyl-dimethylbenzyl-ammonium-chlorid oder -bromid, vorzugsweise C₁₂-C₁₈-Alkyl-dimethylbenzyl-ammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinalinium-chlorid, -bromid oder -monoalkylsulfat; N-(C₁₂-C₁₈-Alkylpolyolaminoformylmethyl) pyridinium-chlorid; N-(C₁₂-C₁₈-Alkyl)-N-methyl-morpholinium-chlorid, -bromid oder -monoalkylsulfat; N-(C₁₂-C₁₈-Alkyl)-N-ethyl-morpholinium-chlorid, -bromid oder -monoalkylsulfat; C₁₆-C₁₈-Alkyl-pentaoxethyl-ammonium-chlorid; Diisobutyl-phenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside in den erfindungsgemäßen Mitteln liegt im Bereich von 1 bis 10 %, bevorzugt 2 bis 7 %, besonders bevorzugt 3 % bis 5 %.

Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronic); Fettsäurealkylolamide (Fettsäureamidpolyethylenglykole); N-Alkyl-, N-Alkoxypolyhydroxyfettsäureamide, Saccharoseester; Sorbitester und Polyglykolether.

Der Gewichtsanteil der nichtionischen Tenside in den erfindungsgemäßen Mitteln liegt im Bereich von 1 bis 20 %, bevorzugt 2 bis 10 %, besonders bevorzugt 3 bis 7 %.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; N-Acylaminoalkyl-N,N-dimethyl-acetobetain, vorzugsweise N-(C₈-C₁₈-Acyl)aminopropyl-N,N-dimethylacetobetain; C₁₂-C₁₈-Alkyl-dimethyl-sulfopropyl-betain; Amphotenside auf Basis Imidazolin (Handelsname: Miranol® , Steinapon® ), vorzugsweise das Natrium-Salz des 1-(β-Carboxy-methyloxyethyl)-1-(carboxymethyl)-2-lauryl-imidazoliniums; Aminoxide, z.B. C₁₂-C₁₈₋Alkyldimethylaminoxid, Fettsäureamidoalkyl-dimethylaminoxid.

Der Gewichtsanteil der amphoteren Tenside in den erfindungsgemäßen Mitteln liegt im Bereich von 0,5 bis 20 %, bevorzugt 1 bis 10 %.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind Laurylsulfat, Laurethsulfat, Cocoamidopropylbetain, Natriumcocoylglutamat, Di-natriumlaurethsulfosuccinat und Cocosfettsäurediethanolamid.

Die erfindungsgemäßen Zubereitungen können außerdem weitere in der Kosmetik gebräuchliche Zusätze wie Überfettungsmittel, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen
Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester, wie Ethylenglycoldistearat, aber auch Fettsäuremonoglykolester in Betracht.
Als Farbstoffe können die für kosmetische Zwecke geeigneten und zugelassenen Substanzen verwendet werden.
Als Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol 934, -940, -941, -956, -980,- 981, -1342, -1382.
Als Verdickungs- und Dispergiermittel besonders geeignet sind Ethylenglykolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl (C₁₂-C₂₂, insbesondere C₁₆-C₁₈)-amidobenzoesäure und deren lösliche Salze, N,N-di(C₁₆-C₁₈)amidobenzoesäure und Derivate.
Die Dispergiermittel werden in Konzentrationen von 0,5 bis 10 Gew.-%, bevorzugt von 0,5 bis 5 Gew.-%, besonders bevorzugt von 1 bis 4 Gew.-%, bezogen auf das fertige Mittel eingesetzt.
Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol und Iso-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol.

Als Trägermaterialien in Betracht kommen pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als fungizide Wirkstoffe können Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole und Naftifine eingesetzt werden.

Um die Affinität des/der erfindungsgemäß eingesetzten Oligoester auf der Haut zu verbessern können kationische Guar Polymere in den Gewichtsmengen 0,01 bis 1,0 %, bevorzugt 0,02 bis 0,25 % eingesetzt werden, wie in WO 97/26854 beschrieben.

Silicone, Polyalkylsiloxane, Polyalkylarylsiloxane, Polyethersiloxan-Copolymere, wie in US 5 104 645 und darin zitierten Schriften beschrieben verbessern noch die pflegende Wirkung der erfindungsgemäßen Mittel. Die erfindungsgemäßen Mittel können mit konventionellen Ceramiden, Pseudoceramiden, Fettsäure-N-alkylpolyhydroxyalkylamide, Cholesterin, Cholesterinfettsäureestern, Fettsäuren, Triglyceriden, Cerebrosiden, Phospholipiden und ähnlichen Stoffen, abgemischt werden.

Die Herstellung der erfindungsgemäßen Mittel erfolgt in an sich bekannter Weise durch Zusammengeben der einzelnen Komponenten und eine - soweit erforderlichder jeweiligen Zubereitungsart angepasste Weiterverarbeitung. Einige dieser vielfältigen möglichen Zubereitungsformen werden in den Ausführungsbeispielen beispielhaft beschrieben.

Mit dem erfindungsgemäßen Einsatz von Polyester in wässrige oder wässrig-alkoholische Körperreinigungsmittel, insbesondere in Duschbädern, Duschgels und Schaumbädern lassen sich Schädigungen der Haut vermeiden, sowie die Schaumbeschaffenheit und die Hautfreundlichkeit der Mittel verbessern.

Die nachfolgenden Beispiele sollen den Gegenstand der Erfindung näher erläutern, ohne ihn darauf einzuschränken.

### Beispiele

### Beispiel 1: Duschgel

| | Komponente | Gew.-% |
|---|---|---|
| 1 | PEG-120 Methyl Glucose Dioleat | 1,25 |
| 2 | Polyquaternium-10 | 0,3 |
| 3 | Glycerin | 2,0 |
| 4 | Polyester 1 | 2,0 |
| 5 | Kokosfettsäure | 2,0 |
| 6 | Genagen LDA | 6,2 |
| 7 | Genagen CAB | 3,0 |
| 8 | Hostapon CLG | 3,6 |
| 9 | Zitronensäure 25 % | 1,05 |
| 10 | Methyldibromoglutaronitril/ Phenoxyethanol | 0,05 |
| 11 | Parfüm | 0,5 |
| 12 | Genapol TSM | 4,0 |
| 13 | E-Wasser | ad 100 |

Komponente 1, 2, 4 und 5 wurden vorgelegt und in ca. 70°C heißem E-Wasser unter Rühren gelöst. Nacheinander wurden 6, 7, 8 und 3 unter Rühren zugegeben und der pH-Wert mit Zitronensäure auf pH 6,0 eingestellt. Durch Zugabe von 10 und 11 wurde das Mittel konserviert und parfümiert und mit dem Trübungsmittel 12 versehen.

### Beispiel 2: Duschbad

| | Komponente | Gew.-% |
|---|---|---|
| 1 | PEG-120 Methyl Glucose Dioleat | 1,25 |
| 2 | Polyquaternium-10 | 0,3 |
| 3 | Glycerin | 2,0 |
| 4 | Polyester 3 | 2,0 |
| 5 | Kokosfettsäure | 2,0 |
| 6 | Genapol LRO | 14,0 |
| 7 | Genagen LDA | 1,9 |
| 8 | Genagen CAB | 1,0 |
| 9 | Hostapon CLG | 1,0 |
| 10 | Zitronensäure 25 % | 1,05 |
| 11 | Methyldibromoglutaronitril/ Phenoxyethanol | 0,05 |
| 12 | Parfüm | 0,5 |
| 13 | Titandioxid | 0,2 |
| 14 | E-Wasser | ad 100 |

Komponente 1, 2, 4 und 5 wurden vorgelegt und in ca. 70°C heißem E-Wasser unter Rühren gelöst. Nacheinander wurden 6, 7, 8, 9 und 3 unter Rühren zugegeben und der pH-Wert mit Zitronensäure auf pH 6,1 eingestellt. Durch Zugabe von 11 und 12 wurde das Mittel konserviert und parfümiert und mit dem Trübungsmittel 13 versehen.

### Beispiel 3: Duschbad

| | Komponente | Gew.-% |
|---|---|---|
| 1 | PEG-120 Methyl Glucose Dioleat | 2,25 |
| 2 | Polyquaternium-10 | 0,3 |
| 3 | Glycerin | 2,0 |
| 4 | Polyester 2 | 5,0 |
| 5 | Kokosfettsäure | 2,0 |
| 6 | Medialan LD | 2,0 |
| 7 | Genapol LRO | . 3,15. |
| 8 | Genagen LDA | 5,4 |
| 9 | Genagen CAB | 3,0 |
| 10 | Hostapon CLG | 3,6 |
| 11 | Zitronensäure 25 % | 1,05 |
| 12 | Methyldibromoglutaronitril/ Phenoxyethanol | 0,05 |
| 13 | Parfüm | 0,5 |
| 14 | Natrium-Styrol-Acrylat-Copolymer/ Natrium-Laurylsulfat/Trideceth-7 | 0,8 |
| 15 | E-Wasser | ad 100 |

Komponente 1, 2, 4 und 5 wurden vorgelegt und in ca. 70°C heißem E-Wasser unter Rühren gelöst. Nacheinander wurden 6, 7, 8, 9, 10 und 3 unter Rühren zugegeben und der pH-Wert mit Zitronensäure auf pH 6,2 eingestellt. Durch Zugabe von 12 und 13 wurde das Mittel konserviert und parfümiert und mit dem Trübungsmittel 14 versehen.

**Chemische Bezeichnung der eingesetzten Handelsprodukte**

| | |
|---|---|
| Genapol TSM | PEG-3 Distearate/ Natriumlaurethsulfat |
| Genagen LDA | Lauroamphodiacetat, Natriumsalz |
| Genagen CAB | Cocosamidopropylbetain |
| Hostapon CLG | Lauroylglutamat, Natriumsalz |
| Medialan LD | Lauroylsarcosinat, Natriumsalz |
| Genapol LRO | Laurethsulfat, Natriumsalz |
| Polyester 1 | ca. 40 mol % Terephthalsäure, |
| | ca. 10 mol % Ethylenglykol, |
| | ca. 10 Mol-% Propylenglykol |
| | ca. 20 Mol-% Polyethylenglykol |
| | ca. 10 Mol% Fettalkoholethoxylat |
| | ca. 10 Mol-% Polyol |
| Polyester 2 | ca. 50 mol % Terephthalsäure, |
| | ca. 25 Mol-% Ethylenglykol, |
| | ca. 20 Mol-% Polyethylenglykol |
| | ca. 5 Mol-% Fettalkoholethoxylat |
| Polyester 3 | ca. 50 mol % Hexandisäure |
| | ca. 40 mol % Propylenglykol, |
| | ca. 10 Mol% Polyethylenglykol |

## Patentansprüche

1. Verwendung von Zusammensetzungen zur Reinigung von Haut, **dadurch gekennzeichnet, dass** es sich bei den Zusammensetzungen um wässrige oder wässrig-alkoholische Körperreinigungsmittel, ausgenommen Haarbehandlungsmittel, handelt, die
Oligoesterenthalten, die durch Kondensation von einer oder mehreren Dicarbonsäuren oder deren Estern und einem oder mehreren mehrwertigen Alkoholen erhalten werden,
5 bis 70 Gew.-% an Tensiden enthalten,
schäumend sind, und
keine W/O- oder O/W-Emulsionen darstellen.

2. Verwendung nach Anspruch 1, **dadurch gekennzeichnet, dass** die Oligoester durch Polykondensation von einer oder mehreren Dicarbonsäuren oder deren Estern und Ethylenglykol und/oder Propylenglykol sowie gegebenenfalls Polypropylenglykol, Polyethylenglykol, Sulfoisophthalsäure, Sulfobenzoesäure, Isethionsäure, C₁-C₄₋Alkohole, oxalkylierte C₁-C₂₄-Alkohole, oxalkylierte C₆-C₁₈-Alkylphenole oder oxalkylierte C₈-C₂₄-Alkylamine erhalten werden.

3. Verwendung nach Anspruch 1, **dadurch gekennzeichnet, dass** die Oligoester durch Polykondensation von
a) 40 bis 52, vorzugsweise 45 bis 50 Mol-% einer oder mehrerer aromatischer Dicarbonsäuren oder deren Ester,
b) 10 bis 60, vorzugsweise 20 bis 35 Mol-% Ethylenglykol und/oder Propylenglykol,
c) 0 bis 20, vorzugsweise 10 bis 15 Mol-% Polyethylenglykol,
d) 0 bis 10 Mol-% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 Mol-% eines oder mehrerer Polyole mit 3 bis 6 Hydroxylgruppen erhalten werden.

4. Verwendung nach Anspruch 1, **dadurch gekennzeichnet, dass** die Zusammensetzungen 0,1 bis 5 Gew.-% Oligoester enthalten.

## Claims

1. The use of compositions for cleansing the skin, wherein the compositions are aqueous or aqueous-alcoholic body-cleansing compositions, with the exception of hair-treatment compositions, which comprise oligoesters obtained by condensation of one or more dicarboxylic acids or esters thereof and one or more polyhydric alcohols.
comprise 5 to 70% by weight of surfactants
are foaming and
do not constitute W/O or O/W emulsions.

2. The use as claimed in claim 1, wherein the oligoesters are obtained by polycondensation of one or more dicarboxylic acids or esters thereof and ethylene glycol and/or propylene glycol, and optionally polypropylene glycol, polyethylene glycol, sulfoisophthalic acid, sulfobenzoic acid,
isethionic acid, C₁-C₄-alcohols, oxalkylated C₁-C₂₄-alcohols, oxalkylated C₆-C₁₈₋alkylphenols or oxalkylated C₈-C₂₄-alkylamines.

3. The use as claimed in claim 1, wherein the oligoesters are obtained by polycondensation of
a) 40 to 52 mol%, preferably 45 to 50 mol%, of one or more aromatic dicarboxylic acids or esters thereof,
b) 10 to 60 mol%, preferably 20 to 35 mol%, of ethylene glycol and/or propylene glycol,
c) 0 to 20 mol%, preferably 10 to 15 mol%, of polyethylene glycol,
d) 0 to 10 mol% of a water-soluble addition product of from 5 to 80 mol of an alkylene oxide with 1 mol of C₁-C₂₄-alcohols, C₆-C₁₈-alkylphenols or C₈-C₂₄-alkylamines and
e) 0 to 10 mol% of one or more polyols having 3 to 6 hydroxyl groups.

4. The use as claimed in claim 1, wherein the compositions comprise 0.1 to 5% by weight of oligoesters.

## Revendications

1. Utilisation de compositions pour le nettoyage de la peau, **caractérisée en ce qu'**il s'agit pour les compositions de compositions nettoyantes aqueuses ou hydro-alcooliques, à l'exception des produits d'entretien des cheveux, qui
contiennent des oligoesters, qui sont obtenus par condensation d'un ou plusieurs acides dicarboxyliques ou de leurs esters avec un ou plusieurs alcools polyhydroxylés,
contiennent 5 à 70 % en poids d'agents tensio-actifs, sont moussants, et
ne forment pas d'émulsions E/H ou H/E.

2. Utilisation selon la revendication 1, **caractérisée en ce qu'**on obtient les oligoesters par polycondensation d'un ou plusieurs acides dicarboxyliques ou de leurs esters avec l'éthylèneglycol et/ou le propylèneglycol ainsi qu'éventuéllement le polypropylèneglycol, le polyéthylèneglycol, l'acide sulfoisophtaliques, l'acide sulfobenzoïque, l'acide iséthionique, les alcools en C₁ à C₄, les alcools en C₁ à C₂₄ alcoxylés, les alkylphénols en C₆ à C₁₈ alcoxylés ou les alkylamines en C₈ à C₂₄ alcoxylées.

3. Utilisation selon la revendication 1, **caractérisée en ce que** l'on obtient les oligoesters par polycondensation de
a) 40 à 52, de préférence 45 à 50 % en moles d'un ou de plusieurs acides dicarboxyliques aromatiques ou de leurs esters,
b) 10 à 60, de préférence 20 à 35 % en moles d'éthylèneglycol et/ou de propylèneglycol,
c) 0 à 20, de préférence 10 à 15 % en moles de polyéthylèneglycol,
d) 0 à 10 % en moles d'un produit de fixation par addition hydrosoluble de 5 à 80 mol d'un oxyde d'alkylène sur 1 mol d'alcools en C₁ à C₂₄, d'alkylphénols en C₆ à C₁₈ ou d' alkylamines en C₈ à C₂₄ et
e) 0 à 10 % en moles d'un ou plusieurs polyols avec 3 à 6 groupes hydroxyle.

4. Utilisation selon la revendication 1, **caractérisée en ce que** les compositions contiennent de 0,1 à 5 % en poids d'oligoester.
